(19) Europäisches Patentamt
European Patent Office
Office européen des brevets

(11) Publication number: 0 453 275 A1

(12) # EUROPEAN PATENT APPLICATION

(21) Application number: 91303452.6

(22) Date of filing: 18.04.91

(51) Int. Cl.⁵: **C07D 301/03**, C07D 303/04, C07D 303/06, C08G 65/02

(30) Priority: 19.04.90 JP 101572/90

(43) Date of publication of application: 23.10.91 Bulletin 91/43

(84) Designated Contracting States: DE FR GB IT

(71) Applicant: NIPPON OIL CO. LTD.
3-12, Nishi Shinbashi 1-chome
Minato-ku Tokyo (JP)

(72) Inventor: Ikai, Keizo
Towa-Garden-House Hayame 506, 887-1, Nagae
Hayama-machi, Miura-gun, Kanagawa-ken (JP)
Inventor: Kobayashi, Masaaki
88-3, Shinohara-cho, Kohoku-ku
Yokohama-shi, Kanagawa-ken (JP)
Inventor: Suzuki, Keisuke
612-2, Kamihirama, Nakahara-ku
Kawasaki-shi, Kanagawa-ken (JP)
Inventor: Matsuno, Mitsuo
12-14, Hino 1-chome, Konan-ku
Yokohama-shi, Kanagawa-ken (JP)

(74) Representative: Myerscough, Philip Boyd et al
J.A. Kemp & Co. 14 South Square, Gray's Inn
London WC1R 5LX (GB)

(54) **Process for producing epoxy compound.**

(57) 6-Epoxyethyl-3-oxatricyclo[3.2.1.$0^{2,4}$]octane derivatives and 2,3-epoxy-6-epoxyethyl-1,4 ;5,8-dimethano-decahydronaphthalene derivatives which are expected to be used as monomers to give plastics having a high transition temperature are easily produced by oxidizing corresponding 6-vinyl compounds with dioxorane.

EP 0 453 275 A1

## FIELD OF THE INVENTION

This invention relates to a process for producing a novel diepoxy compound using a dioxirane compound.

## PRIOR ART AND PROBLEMS THE INVENTION ATTEMPTS TO SOLVE

Since polyether compounds obtained by ring opening polymerization of 6-epoxyethyl-3-oxatricyclo-[3.2.1.0$^{2,4}$]octane or 2,3-epoxy-6-epoxyethyl-1,4:5,8-dimethanodecahydronaphthalene have a bicyclo[2.2.1]-heptane skeleton or a dimethanonaphthalene skeleton, they are amorphous, have a high glass transition temperature, and are excellent in transparency and water resistance and free from impurities such as chlorine, etc.; their use as plastics having excellent electrical characteristics has been expected. For example, use of said compounds as sealing epoxy resins of VLSI or printed circuit boards, or their optical use has been expected. Moreover, use of the monomers as new reactive epoxy diluents has been also expected.

Industrial production of these compounds is however difficult. For example, synthesis of 6-epoxyethyl-3-oxatricyclo[3.2.1.0$^{2,4}$]octane requires costly peracids such as peracetic acid, perbenzoic acid, etc., and its yield is as low as about 50 % [L. I. Kasyan, M.F. Bombushkary, M. S. Malinobsky et al., Ukr. Khim. Zh., 44, 956 (1978)].

## MEANS TO SOLVE THE PROBLEMS

The present inventors have made assiduous studies to solve such problems, and consequently found a process in which a compound represented by the following formula (IV) is formed from an easily available, inexpensive peroxide and a compound represented by the following formula (I) and/or a compound represented by the following formula (II) effectively at low cost. This finding has led to the completion of this invention.

It is an object of this invention to provide a novel process for producing a compound represented by the following formula (IV) using a dioxirane compound.

## DETAILED DESCRIPTION OF THE INVENTION

This invention relates to a process for producing a diepoxy compound represented by formula (IV)

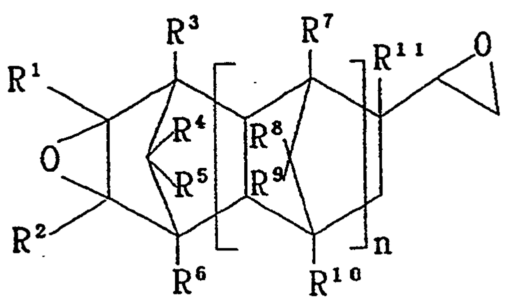

(IV)

wherein $R^1$, $R^2$, $R^3$, $R^4$, $R^5$, $R^6$, $R^7$, $R^8$, $R^9$, $R^{10}$ and $R^{11}$ each denote a hydrogen atom or a hydrocarbon group, and n is an integer of 0 to 2,
which comprises epoxidizing a compound represented by formula (I)

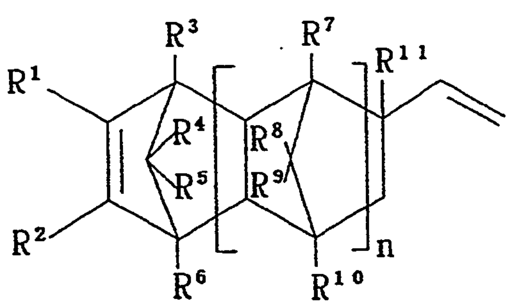

(I)

wherein $R^1$, $R^2$, $R^3$, $R^4$, $R^5$, $R^6$, $R^7$, $R^8$, $R^9$, $R^{10}$, $R^{11}$ and n are as defined above,

and/or a compound represented by formula (II)

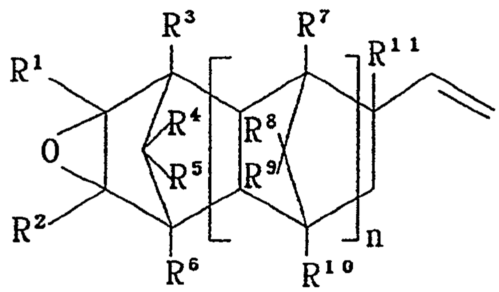

(II)

wherein $R^1$, $R^2$, $R^3$, $R^4$, $R^5$, $R^6$, $R^7$, $R^8$, $R^9$, $R^{10}$, $R^{11}$ and n are as defined above, with a dioxirane compound represented by formula (III)

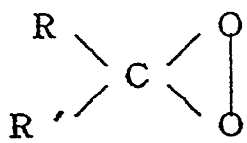

(III)

wherein R and R′ each denote a hydrogen atom, a fluorine atom, an alkyl group having 1 to 10 carbon atoms, an aryl group, a fluoroalkyl group or a fluoroaryl group.

This invention will be explained in detail below.

In formula (I), n is an integer of 0 to 2, preferably 0 to 1, and $R^1$, $R^2$, $R^3$, $R^4$, $R^5$, $R^6$, $R^7$, $R^8$, $R^9$, $R^{10}$ and $R^{11}$ are each a hydrogen atom or a hydrocarbon group and may be the same or different. Examples of the hydrocarbon group are alkyl groups having usually 1 to 3, preferably 1 to 2 carbon atoms. Examples of the compound of formula (I) include 5-vinylbicyclo[2.2.1]hept-2-ene, 1-methyl-5-vinylbicyclo[2.2.1]hept-2-ene, 1-methyl-6-vinylbicyclo[2.2.1]hept-2-ene, 2-methyl-5-vinylbicyclo-[2.2.1]hept-2-ene, 2-methyl-6-vinylbicyclo [2.2.1]hept-2-ene, 5-methyl-5-vinylbicyclo[2.2.1]hept-2-ene, 1-ethyl-6-vinylbicyclo[2.2.1]hept-2-ene, 2-vinyl-1, 2,3,4,4a,5,8,-8a-octahydro-1,4:5,8-dimethanonaphthalene, and 12-vinylhexacyclo[6.6.1.$1^{3,6}$.$1^{10,13}$.$0^{2,7}$.$0^{9,14}$]-4-heptadecene.

In formula (II), n is an integer of 0 to 2, preferably 0 to 1, and $R^1$, $R^2$, $R^3$, $R^4$, $R^5$, $R^6$, $R^7$, $R^8$, $R^9$, $R^{10}$ and $R^{11}$ are each a hydrogen atom or a hydrocarbon group and may be the same or different. Examples of the hydrocarbon group are alkyl groups having usually 1 to 3, preferably 1 to 2 carbon atoms. Examples of the compound of formula (II) include 6-vinyl-3-oxatricyclo-[3.2.1.$0^{2,4}$]octane, 1-methyl-6-vinyl-3-oxatricyclo-[3.2.1.$0^{2,4}$] octane, 1-methyl-7-vinyl-3-oxatricyclo-[3.2.1.$0^{2,4}$]octane, 2-methyl-6-vinyl-3-oxatricyclo-[3.2.1.$0^{2,4}$]octane, 2-methyl-7-vinyl-3-oxatricyclo-[3.2.1.$0^{2,4}$]octane, 6-methyl-6-vinyl-3-oxatricyclo-[3.2.1.$0^{2,4}$]octane, 1-ethyl-7-vinyl-3-oxatricyclo-[3.2.1.$0^{2,4}$]octane, 2,3-epoxy-6-vinyl-1,4:5,8- dimethano-1,2,3,4,4a,5,8,8a-octahydronaphthalene, and 4,5-epoxy-12-vinylhexacyclo[6.6.1.$1^{3,6}$.$1^{10,13}$.$0^{2,7}$.$0^{9,14}$]-heptadecane.

In formula (III), R and R′ are each a hydrogen atom, a fluorine or an alkyl group having 1 to 10, preferably 1 to 6 carbon atoms, an aryl group, a fluoroalkyl group or a fluoroaryl group. Examples of the compound of formula (III) include dioxirane, methyldioxirane, ethyldioxirane, pentyldioxirane, hexyldioxirane, heptyldioxirane, octyldioxirane, phenyldioxirane, difluorodioxirane, dimethyldioxirane, ethylmethyldioxirane, methylpropyldioxirane, methylbutyldioxirane, methylpentyldioxirane, methylhexyldioxirane, methylheptyldioxirane, diethyldioxirane, ethylpropyldioxirane, ethylbutyldioxirane, ethylpentyldioxirane, ethylhexyldioxirane, ethylheptyldioxirane, dipropyldioxirane, hexylpropyldioxirane, dibutyldioxirane, methylphenyldioxirane, diphenyldioxirane, methyltrifluoromethyldioxirane, ditrifluoromethyldioxirane, and fluoromethylphenyldioxirane.

A method of forming the compound of formula (III) is not particularly limited. Said compound is usually formed by the reaction with an aldehyde or a ketone and an inexpensive inorganic peroxide.

As the aldehyde and the ketone, compounds represented by formula (V) are shown.

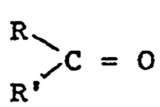

(V)

wherein R and R' are as defined in formula (IV).

Examples thereof include formaldehyde, acetaldehyde, propionaldehyde, caproaldehyde, heptylaldehyde, octylaldehyde, nonylaldehyde, benzaldehyde, carbonic difluoride, acetone, 2-butanone, 2-pentanone, 2-hexanone, 2-heptanone, 2-octanone, 2-nonanone, 2-decanone, 3-pentanone, 3-hexanone, 3-heptanone, 3-octanone, 3-nonanone, 3-decanone, 4-heptanone, 4-decanone, 5-nonanone, acetophenone, benzophenone, trifluoroacetone, hexafluoroacetone, and trifluoroacetophenone. Examples of the inorganic peroxide include $KHSO_5$, $2KHSO_5 \cdot KHSO_4 \cdot K_2SO_4$, $H_2SO_5$, $NaIO_4$, $KIO_4$ and double salts substantially containing these peroxides. $KHSO_5$ or $H_2SO_5$ can be formed from $K_2S_2O_8$.

The reaction with the aldehyde or the ketone and the inorganic peroxide can be carried out in a known manner. Namely, the reaction is performed by mixing the aldehyde or the ketone with the aqueous solution of the inorganic peroxide. On this occasion, various quaternary ammonium salts and crown ethers may be used as a phase-transfer catalyst. Examples of the quaternary ammonium salts include tetraethylammonium bromide, tetraethylammonium hydrogensulfate, tetrabutylammonium bromide, and tetrabutylammonium hydrogensulfate. Examples of the crown ether include 12-crown-4-, 15-crown-5, 18-crown-6, dibenzo-14-crown-4, dibenzo-15-crown-5, and dibenzo-18-crown-6. It is advisable that the reaction is performed at pH of usually 4.0 to 8.0, preferably 6.0 to 8.0, more preferably 7.5 to 8.0. The pH is adjusted in a usual manner. That is, the pH adjustment is carried out using a buffer solution while adding an alkali aqueous solution during the reaction. Examples of the alkali in the alkali aqueous solution include sodium hydroxide, potassium hydroxide and calcium hydroxide. Examples of the buffer solution include potassium dihydrogenphosphate-sodium hydroxide, potassium dihydrogenphosphate-disodium hydrogenphosphate, potassium dihydrogenphosphate-sodium tetraborate, boric acid+sodium chloride-sodium tetraborate, and disodium hydrogenphosphate-citric acid. At this time, when the reaction temperature is too high, decomposition of the resulting dioxirane compound occurs. When the reaction temperature is too low, the reaction is delayed. Therefore, it is usually -20 to 30°C, preferably 5 to 15°C. The reaction time is usually 1 to 20 hours, preferably 5 to 10 hours. A molar ratio of the inorganic peroxide to the aldehyde or the ketone is 0.1 to 1, preferably 0.5 to 1. The resulting dioxirane compound is isolated by a known method, e.g. distillation under reduced pressure. At this time, the unreacted aldehyde or ketone may be distilled off together.

In this invention, as stated above, the compound of formula (IV) is produced by epoxidizing the compound of formula (I) and/or the compound of formula (II) (hereinafter abbreviated as "olefins") with the dioxirane compound of formula (III).

The epoxidation reaction may be conducted either simultaneously while forming the dioxirane compound, or after isolating the resulting dioxirane compound as noted above. The former is preferable.

When the epoxidation reaction is conducted simultaneously with the formation of the dioxirane compound, the mixture of the olefins and the aldehyde or the ketone is mixed with the aqueous solution of hte inorganic peroxide. The reaction is carried out in the same way as in forming the aforesaid dioxirane compound. The phase-transfer catalyst may be used or an organic solvent may not be used. The organic solvent is not particularly limited if it is inactive to the dioxirane compound. Examples of the organic solvent include hexane, cyclohexane, toluene, xylene, dichloromethane, chloroform and carbon tetrachloride. It is advisable to conduct the reaction at pH of usually 4.6 to 8.0, preferably 6.5 to 8.0, more preferably 7.5 to 8.0. The pH can be adjusted in a usual manner as in producing the dioxirane compound. The reaction temperature is usually -5 to 30°C, preferably 5 to 15°C. The reaction time is usually 1 to 20 hours, preferably 5 to 10 hours. A molar ratio of the aldehyde or the ketone to the olefins is usually 0.5 to 5, preferably 1 to 2. A molar ratio of the inorganic peroxide to the olefins is usually 1 to 5, preferably 1 to 2.

When the epoxidation is carried out using the isolated dioxirane compound, the reaction is run by mixing the dioxirane compound with the olefins. A molar ratio of the dioxirane compound to the olefins is usually 1.0 to 4.0, preferably 1.0 to 2.0. The reaction temperature is usually -20 to 30°C, preferably 5 to 15°C. The reaction time is usually 1 to 20 hours, preferably 5 to 10 hours. On this occasion, an organic solvent may or may not be used. When the organic solvent is used, it is not particularly limited if it is inactive to the dioxirane. Examples of the organic solvent include hexane, cyclohexane, toluene, xylene, dichloromethane, chloroform and carbon tetrachloride.

After the reaction is terminated, the post-treatment is carried out by a usual method such as distillation, etc., and the organic layer is separated and purified to obtain the final compound of formula (IV).

When the compound of formula (I) is epoxidized with hydrogen peroxide or hydroperoxides such as tertbutyl hydroperoxide, etc., a double bond in a ring is easily epoxidized to form a compound of formula (II). However, a double bond of a vinyl group is low in reactivity; before this is epoxidized, the addition reaction of the hydroperoxides or hydroxides occurs on the compound (II), and the final compound of formula (IV) is little formed. To obtain the compound (IV), a reagent high in reactivity and capable of epoxidation under mild conditions, such as m-chlorobenzoic acid, is required, but such peracid is pricey and impractical.

This is the same with the epoxidation of the compound of formula (II).

In the epoxidation reaction using the dioxirane compound (III), the reaction conditions are quite mild and reactivity is high.

[EFFECTS OF THE INVENTION]

The process of this invention has excellent characteristics that the compound of formula (IV) can be synthesized efficiently at low cost from starting materials that can be obtained easily at low cost, and can efficiently provide the compound of formula (IV) that is utilizable as a starting material of plastics excellent in heat resistance, water resistance, transparency and electrical characteristics, and as a new epoxy diluent.

[EXAMPLES]

The following Examples and Comparative Example illustrate this invention specifically. However, this invention is not limited thereto.

EXAMPLE 1

A 3-liter three-necked flask fitted with a stirrer, a pH electrode and a thermometer was charged with 43 g (0.36 mol) of 5-vinylbicyclo[2.2.1]hept-2-ene, 400 ml of methylene chloride, 200 ml of acetone, 280 ml of a 0.05 M phosphoric acid buffer solution (pH 7.4) and 19 g (56 mmols) of tetrabutylammonium hydrogensulfate. While cooling the mixture to 10°C, an aqueous solution (water, 2,200 ml) of 555 g (1.81 mols as $KHSO_5$) of Oxone ($2KHSO_5.KHSO_4.K_2SO_4$ made by du Pont) was added dropwise at a rate of 10 ml/min under stirring. During the reaction, a 5N KOH aqueous solution was added dropwise using a pH controller to adjust pH to 7.2 to 7.8. After the addition of the Oxone aqueous solution, stirring further continued for 4 hours. The reaction solution was suction-filtered with a Buchner funnel, and $K_2SO_4$ precipitated was separated by filtration. A methylene chloride layer was then separated. Said methylene chloride layer was dried with magnesium sulfate to distill off methylene chloride. The concentrate was passed through a silica gel column to remove tetrabutylammonium hydrogensulfate. Subsequently, the residue was subjected to single distillation under reduced pressure to obtain 46 g of 6-epoxyethyl-3-oxatricyclo[3.2.1.$0^{2,4}$]octane (yield 85 %).

EXAMPLE 2

In the same way as in Example 1, while keeping pH at 7.2 to 7.6, 2,000 ml of an aqueous solution (water) of 450 g (1.46 mols as $KHSO_5$) of Oxone ($2KHSO_5.KHSO_4.-K_2SO_4$ made by du Pont) was added to a mixture of 100 g (0.734 mol) of 6-vinyl-3-oxatricyclo[3.2.1.$0^{2,4}$]octane, 120 ml of acetone, 200 ml of a 0.05M phosphoric acid buffer solution (pH 7.4) and 13 g (37 mmols) of tetrabutylammonium hydrogensulfate at a rate of 10 ml/min under cooling to 10°C with stirring. After the addition, stirring further continued for 6 hours. The reaction solution was suction-filtered with a Buchner funnel, and $K_2SO_4$ precipitated was separated by filtration, followed by extracting the filtrate with toluene (500 ml x 1 + 300 ml x 3). The toluene layer was passed through a column of a molecular sieve (4A), dried and concentrated. The concentrate was subjected to single distillation under reduced pressure to obtain 84 g of 6-epoxyethyl-3-oxatricyclo[3.2.1.$0^{2,4}$]octane (yield 75 %).

EXAMPLE 3

In the same way as in Example 1, while keeping pH at 7.2 to 7.6, 1,400 ml of an aqueous solution (water) of 300 g of Oxone ($2KHSO_5.KHSO_4.K_2SO_4$ made by du Pont) was added dropwise to a mixture of 45 g (0.242 mol) of 2-vinyl-1,2,3,4,4a,5,8,8a-octahydro-1,4;5,8-dimethanonaphthalene, 300 ml of methylene chloride, 150 ml of acetone, 200 ml of a 0.05M phosphoric acid buffer solution (pH 7.4) and 8 g (24 mmols) of tetrabutylammonium hydrogensulfate under cooling to 10°C with stirring. After the addition, stirring further continued for 6 hours. The reaction solution was suction-filtered with a Buchner funnel, and $K_2SO_4$ precipitated was separated by filtration. Subsequently, the filtrate was extracted with toluene (500 ml x 1 + 300 ml x 3). The toluene layer was passed through a column of a molecular sieve (4A), dried and concentrated to obtain 50 g of 2,3-epoxy-6-epoxyethyl-1,4;5,8-dimethano-decahydronaphthalene (yield 94 %).

COMPARATIVE EXAMPLE 1

To a mixture of 6.8 g (0.050 mol) of 6-vinyl-3-oxatricyclo[3.2.1.$0^{2,4}$]octane and 0.16 g (0.5 mol) of $MoO_2$-

$(acac)_2$ was added 5.6 g (0.050 mol) of 80 % tert.-butyl hydroperoxide at room temperature under stirring. After the addition, the reaction continued at 50°C for 10 hours. Analysis of the reaction solution by gas chromatography revealed that the yield of 6-epoxyethyl-3-oxatricyclo[3.2.1.$0^{2,4}$]octane as a final compound was 3 % and unreacted 6-vinyl-3-oxatricyclo[3.2.1.$0^{2,4}$]octane was 10 %.

## Claims

1. A process for producing a diepoxy compound represented by formula (IV)

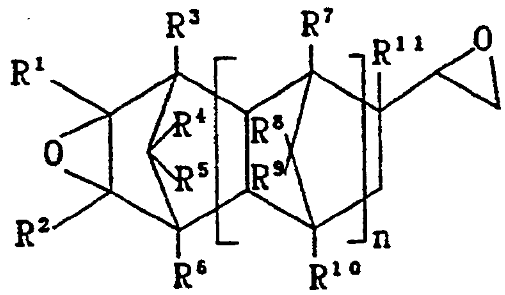

(IV)

wherein $R^1$, $R^2$, $R^3$, $R^4$, $R^5$, $R^6$, $R^7$, $R^8$, $R^9$, $R^{10}$ and $R^{11}$, which may be the same or different, each represent a hydrogen atom or a hydrocarbon group, and n is an integer of 0 to 2, which process comprises epoxidizing a compound represented by formula (I)

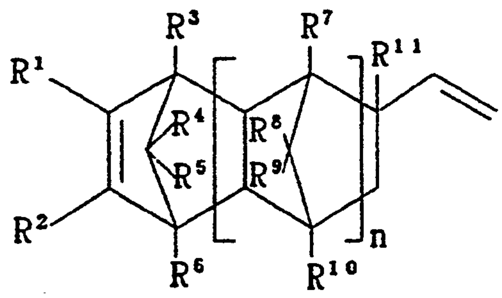

(I)

wherein $R^1$, $R^2$, $R^3$, $R^4$, $R^5$, $R^6$, $R^7$, $R^8$, $R^9$, $R^{10}$, $R^{11}$ and n are as defined above, and/or a compound represented by formula (II)

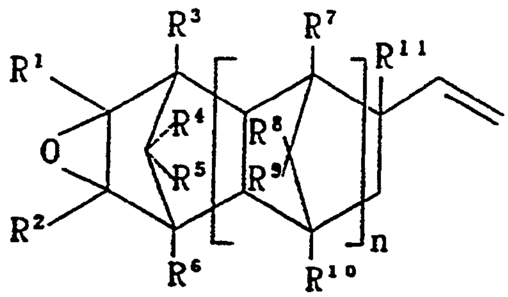

(II)

wherein $R^1$, $R^2$, $R^3$, $R^4$, $R^5$, $R^6$, $R^7$, $R^8$, $R^9$, $R^{10}$, $R^{11}$ and n are as defined above, with a dioxirane compound represented by formula (III)

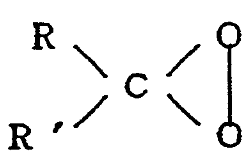

(III)

wherein R and R′, which may be the same or different, each represent a hydrogen atom, a fluorine atom, an alkyl group having 1 to 10 carbon atoms, an aryl group, a fluoroalkyl group or a fluoroaryl group.

2. A process according to claim 1 wherein the compound of formula (I) is 5-vinylcyclo[2.2.1]hept-2-ene, 1-methyl-5-vinylbicyclo[2.2.1]hept-2-ene, 1-methyl-6-vinylbicyclo-[2.2.1]hept-2-ene, 2-methyl-5-vinylbicyclo [2.2.1]hept-2-ene, 2-methyl-6-vinylbicyclo[2.2.1]hept-2-ene, 5-methyl-5-vinylbicyclo[2.2.1]hept-2-ene, 1-ethyl-6-vinylbicyclo-[2.2.1]hept-2-ene, 2-vinyl-1,2,3,4,4a,5,8,8a-octahydro-1,4:5,8-dimethanonaphthalene or 12-vinylhexacyclo-[6.6.1.$1^{3,6}$.$1^{10,13}$.$0^{2,7}$.$0^{9,14}$]-4-heptadecene.

3. A process according to claim 1 wherein the compound of formula (II) is 6-vinyl-3-oxatricyclo[3.2.1.$0^{2,4}$]octane, 1-methyl-6-vinyl-3-oxatricyclo[3.2.1.$0^{2,4}$]octane, 1-methyl-7-vinyl-3-oxatricyclo[3.2.1.$0^{2,4}$]octane, 2-methyl-6-vinyl-3-oxatricyclo[3.2.1.$0^{2,4}$]octane, 2-methyl-7-vinyl-3-oxatricyclo[3.2.1.$0^{2,4}$]octane, 6-methyl-6-vinyl-3-oxatricyclo[3.2.1.$0^{2,4}$]octane, 1-ethyl-7-vinyl-3-oxatricyclo[3.2.1.$0^{2,4}$]octane, 2,3-epoxy-6-vinyl-1,4:5,8-dimethano-1,2,3,4,4a,5,8,8a-octahydronaphthalene or 4,5-epoxy-12-vinylhexacyclo-[6.6.1.$1^{3,6}$.$1^{10,13}$.$0^{2,7}$.$0^{9,14}$]heptadecane.

4. A process according to claim 1, 2 or 3 wherein the compound of formula (III) is dioxirane, methyldioxirane, ethyl-dioxirane, pentyldioxirane, hexyldioxirane, heptyldioxirane, octyldioxirane, phenyldioxirane, difluorodioxirane, dimethyldioxirane, ethylmethyldioxirane, methylpropyldioxirane, methylbutyldioxirane, methylpentyldioxirane, methylhexyldioxirane, methylheptyldioxirane, diethyldioxirane, ethylpropyldioxirane, ethylbutyldioxirane, ethylpentyldioxirane, ethylhexyldioxirane, ethylheptyldioxirane, dipropyldioxirane, hexylpropyldioxirane, dibutyldioxirane, methylphenyldioxirane, diphenyldioxirane, methyltrifluoromethyldioxirane, ditrifluoromethyldioxirane or trifluoromethylphenyldioxirane.

5. A process according to any one of the preceding claims wherein the compound of formula (III) is formed in situ by the reaction of an aldehyde or a ketone with an inorganic peroxide.

6. A process according to claim 5 wherein the aldehyde or the ketone is formaldehyde, acetaldehyde, propionaldehyde, caproaldehyde, heptylaldehyde, octylaldehyde, nonylaldehyde, benzaldehyde, carbonic difluoride, acetone, 2-butanone, 2-pentanone, 2-hexanone, 2-heptanone, 2-octanone, 2-nonanone, 2-decanone, 3-pentanone, 3-hexanone, 3-heptanone, 3-octanone, 3-nonanone, 3-decanone, 4-heptanone, 4-decanone, 5-nonanone, acetophenone, benzophenone, trifluoroacetone, hexafluoroacetone or trifluoroacetophenone.

7. A process according to claim 5 or 6 wherein the inorganic peroxide is $KSO_5$, $2KSO_5 . KSO_4 . K_2SO_4$, $H_2SO_5$, $NaIO_4$, $KIO_4$ or a double salt containing these peroxides.

8. A process according to any one of the preceding claims wherein the compound of formula (IV) is 6-epoxyethyl-3-oxatricyclo-[3.2.1.$0^{2,4}$]octane or 2,3-epoxy-6-epoxyethyl-1,4;5,8-dimethanodecahydronaphthalene.

9. A process according to any one of the preceding claims which comprises the additional step of ring-opening polymerising the diepoxy compound of formula (IV) to produce a polyether.

European Patent Office

# EUROPEAN SEARCH REPORT

Application Number

EP 91 30 3452

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | JOURNAL OF ORGANIC CHEMISTRY, vol. 45, no. 23, 7th November 1980, pages 4758-4760, Easton, US; R. CURCI et al.: "Epoxidation of alkenes by dioxirane intermediates generated in the reaction of potassium caroate with ketones" * The whole article * | 1-9 | C 07 D 301/03<br>C 07 D 303/04<br>C 07 D 303/06<br>C 08 G 65/02 |
| Y | JOURNAL OF ORGANIC CHEMISTRY, vol. 50, no. 16, 9th August 1985, pages 2847-2853, Easton, US; K.W. MURRAY et al.: "Dioxiranes: Synthesis and reactions of methyldioxiranes" * The whole article * | 1-9 | |
| Y | US-A-3 183 249 (H.K. WIESE) * The whole document * | 1-9 | |
| Y | JOURNAL OF ORGANIC CHEMISTRY, vol. 53, no. 13, 24th June 1988, pages 3007-3011, Easton, US; R.W. MURRAY et al.: "Chemistry and dioxiranes. 10. Oxidation of quadricyclane and norbornadiene by dimethyldioxirane" * The whole article * | 1-9 | TECHNICAL FIELDS SEARCHED (Int. Cl.5)<br>C 07 D 301/00<br>C 07 D 303/00 |

The present search report has been drawn up for all claims

| Place of search | Date of completion of the search | Examiner |
|---|---|---|
| THE HAGUE | 18-07-1991 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)